# EUROPEAN PATENT SPECIFICATION

(11) **EP 0 581 844 B1**
(45) Date of publication and mention of the grant of the patent: **25.06.1997**
(21) Application number: 92909927.3
(22) Date of filing: 23.03.1992
(51) Int. Cl.: A61K 7/40, A61K 31/79, C01B 15/037

(54) **PROCESS FOR PRODUCING PVP-H2O2 PRODUCTS**
VERFAHREN ZUR HERSTELLUNG VON PVP-H202-PRODUKTEN
PROCEDE DE PRODUCTION DE PRODUITS EN PVP-H2O2

(30) Priority: 08.04.1991 US 683467; 26.06.1991 US 721786; 04.03.1992 US 883617
(43) Date of publication of application: 09.02.1994
(73) Proprietor: ISP INVESTMENTS INC., Wilmington, Delaware 19801 (US)
(72) Inventor: BISS, Russel, B., Wayne, NJ 07470 (US); COHEN, Jeffrey, M., Hawthorne NJ 07506 (US); MERIANOS, John, J., Middletown, NJ 07748 (US); TAYLOR, Paul, D., Dublin OH 43017 (US)
(74) Representative: Ford, Michael Frederick
(86) International application number: US9202287
(87) International publication number: WO9217158

(56) References cited:
- US-A- 3 480 557
- US-A- 3 908 045
- US-A- 5 008 093
- US-A- 5 008 106

## Description

This invention relates to a process for making a polyvinylpyrrolidone-hydrogen peroxide (PVP-H₂O₂) product, and more particularly, to a fluidized bed process for the production of such material in the form of free-flowing powders or a free-standing, porous solid of foam.

Stabilized H₂O₂ compositions have found wide utility in commercial and industrial applications, e.g. as antiseptics, disinfectants, sterilization agents, bleaching materials, washing concentrates, etchants, in cosmetic preparations, and as a catalyst in polymerizations requiring a free radical source. In biological applications which require an antiseptic, disinfectant or sterilization agent, such H₂O₂ compositions require release of an effective amount of oxygen at a desired rate.

Shiraeff, in US-A-3,376,110 and 3,480,557, disclosed that a solid, stabilized hydrogen peroxide composition of hydrogen peroxide and a polymeric N-vinyl heterocyclic compound could be prepared from an aqueous solution of the components. The process involved mixing PVP and a substantial excess of aqueous H₂O₂ and evaporating the solution to dryness. The H₂O₂ content of the composition was given as being at least 2%, and preferably 4.5 to 70% by weight. Prolonged drying of the composition, in an attempt to reduce the water content, however, resulted in a substantial loss of H₂O₂ from the complex. The product was a brittle, transparent, gummy, amorphous material, and had a variable H₂O₂ content ranging from about 3.20 to 18.07% by weight, depending upon the drying times.

The Shiraeff process did not attain commercial success because (1) the product was not a free-flowing powder and thus could not be handled easily; (2) its water and hydrogen peroxide content varied over a wide range; (3) the complex was not stable; and (4) the aqueous process could not be carried out on a commercial scale.

Rossberger et al., in DE-A-3,444,552, published June 12, 1986, described a process for making urea peroxyhydrate using a fluidized bed of urea having a particle size of less than 1000 µm onto which was sprayed an aqueous solution of concentrated H₂O₂. This technique was practical only because urea is a stable, crystalline compound which readily formed a free-flowing powdery complex upon addition of aqueous hydrogen peroxide solutions.

Production of free-flowing or foam complexes of PVP-H₂O₂ from amorphous, polymeric, polyvinylpyrrolidone and aqueous H₂O₂, however, has not been easy to achieve commercially because, during production, the PVP polymer can alter its physical state, and/or retain excess water and/or free H₂O₂, even at elevated drying temperatures, resulting in a gummy rather than a free-flowing of foam product.

The invention is as defined in the claims.

The invention provides a process for the production of PVP-H₂O₂ products in the form of free-flowing powders in which a fluidized bed of PVP powders maintained at a reaction temperature of from ambient temperature to 60°C. is contacted with finely divided droplets of a 30 to 85% by weight aqueous H₂O₂ solution.

With gelling agent present, e.g. hydroxyethyl cellulose in an amount of about 20-60%, the bulk density and porosity is substantially lower.

The bed temperature is maintained at about ambient temperature to 60°C., preferably 35°-45°C., a 5 to 70% aqueous H₂O₂ solution is used, and the feed rate for introduction of the H₂O₂ solution is about 1-50 g/minute/kg PVP used, preferably 5-25 g/minute/kg PVP.

The free-flowing powder has about 15 to 24% by weight, preferably about 18-22%, H₂O₂ therein, which corresponds to a 1:1 molar ratio of its components, with no excess or free peroxide therein, and having less than about 5% water, which is about the same amount of water originally present in the PVP.

This invention also provides PVP-H₂O₂ complexes in the form of a free-standing, porous, hard solid or foam comprising an integral network of PVP molecules which are hydrogen-bonded to H₂O₂ molecules, optionally including up to 60% by weight of a water-soluble gelling agent, e.g. hydroxyethyl cellulose. The PVP content of the complex without gelling agent suitably is about 70-90% by weight of the product, preferably 75-85%; the H₂O₂ content is about 10-24% by weight, preferably 15-20%, and the water content being less than 10% by weight, preferably, less than 7-1/2%, and, suitably, where the total amount of H₂O₂ and water therein is 25% or less by weight of the product. With gelling agent present, PVP is 25-90%, and H₂O₂ is 5-24%.

The foam products are made by a freeze-dry process using an aqueous PVP-H₂O₂ solution, without gelling agent, suitably comprising about 4-10% by weight H₂O₂, about 15-25% by weight PVP and about 65-85% water. During the freeze-drying step, water is removed from the aqueous PVP-H₂O₂ solution at a temperature below -20°C. and at a reduced pressure of less than about 0.1 mm.

As a foam, the product without gelling agent has a bulk density of about 0.3 to about 0.5 g/cc and a porosity of about 60-70%.

In accordance with the present invention, a fluidized bed containing a charge of PVP powders is reacted with an aqueous solution of concentrated hydrogen peroxide. The PVP polymer can be obtained from GAF Chemicals Corporation in the form of a water-soluble or water-insoluble polymer, which has a molecular weight ranging from the K-15 to K-90 designations. These PVP polymers, which generally have a water content of about 5% by weight, or less, and a particle size of about 10 to 100 microns, may be used directly in the process of the invention, or pre-dried, if desired, to reduce its moisture content.

The hydrogen peroxide solution used herein usually contains about 30 to 85% hydrogen peroxide. A 50 to 70% H₂O₂ solution, however, is preferred because of the inherent danger present in use of the higher H₂O₂ concentrations.

The fluidized bed of PVP powders can be maintained in the fluidized condition by directing a current of dry air through the powders, by mechanical agitation of the powders, or by a combination of both techniques.

The fluidized bed is maintained at a suitable bed (reaction) temperature at which formation of the desired PVP-H₂O₂ complex product can occur readily without affecting the powdery state of the PVP polymer, and at which excess water from the H₂O₂ solution can be quickly removed both from the product and the PVP bed itself. The selected bed temperature also will enhance the formation of a free-flowing powder rather than a gum. Such suitable reaction temperatures range from about ambient temperature to 60°C., preferably about 35° to 45°C.

The aqueous concentrated H₂O₂ solution preferably is contacted with the PVP powders as finely divided droplets of liquid. Such desired droplets may be formed by pumping the H₂O₂ solution through a spray nozzle and onto the PVP bed at a selected rate and for a predetermined period of time. Any spray nozzle capable of producing a fine dispersion of droplets may be used for this purpose. If necessary, however, a stream of air may be introduced into such nozzle with the solution to assist in atomizing the solution into finely divided droplets.

The spray solution of aqueous H₂O₂ thus formed preferably is introduced into the fluidized bed of PVP powders at a selected rate such that excess water can be removed therein during formation of the complex without retaining free H₂O₂ therein. A suitable feed rate for introduction of the H₂O₂ solution is about 5-50 g/minute/kg PVP. Under these flow rate conditions, a free-flowing powder of the desired PVP-H₂O₂ complex is obtained containing about 15-24% H₂O₂, preferably 18-22%, and about 5% or less water therein.

In the preferred form of the process of the invention, the spray solution of H₂O₂ is directed onto the PVP bed for a period sufficient to form a free-flowing powder having an H₂O₂ content of about 15 to 24% by weight, preferably 18-22%, which is indicative of a complex having a 1:1 molar ratio of PVP to H₂O₂. At this point in the process, the feed is discontinued to preclude excess water and/or free H₂O₂ from forming on the free-flowing powder which can cause it to become gummy. The appearance of a gummy product is indicative of the presence of excess water and/or free H₂O₂ in the product.

The spray solution of H₂O₂ may be directed onto the fluidized bed as a vertical, horizontal or by downward flow of droplets.

If a fluidizing air stream is used to create the fluid bed, it is usually directed upwardly against the PVP powders. Such air currents also can assist in carrying water away from the bed. The fluidized state of the bed also may be maintained using mechanical agitation, or a combination of both air and mechanical means.

The process of the invention can be carried out in one or two steps, i.e. removal of water from the product and bed can take place either (a) simultaneous with or after mixing of the reaction components in the same apparatus,or (b) in a downstream drying step, or (c) by a combination of both steps. The particular method of drying will depend upon the type of equipment used. For example, if a fluidized bed mixer is used, such as a plowshare, belt screw or paddle mixer, then the moist PVP-H₂O₂ product can be dried further in a separate dryer. This sequence is characterized as a two-step process. Any suitable dryer can be used for this purpose, such as a vacuum, radiant heat or contact dryer.

Furthermore, if desired, application of the spray H₂O₂ solution onto the PVP bed, followed by downstream drying, may be carried out in several stages in order to increase the H₂O₂ content of the product towards the desired 1:1 molar ratio, and to reduce its water content.

Moreover, a fluid bed dryer may be used in the process which has the dual capabilities of providing both the fluidized bed and drying functions. Accordingly, drying of the product will begin and be completed during reaction between the PVP charge and the aqueous H₂O₂ solution. Such a process may be considered as taking place in a one-step.

Preferably, reaction and dehydration are continued until the product reaches a desired H₂O₂ content, suitably about 15-24% H₂O₂, and usually about 18-22%, with less than about 5% water, generally about 1-3%. However, it is essential that the product remain in the free-flowing state after completion of addition of the H₂O₂ solution.

The size of the fluidized bed reactor, the rate of addition of the hydrogen peroxide solution, and the reaction times will vary with the particular equipment used, as well as the concentration of the hydrogen peroxide solution and the reaction temperature, keeping in mind the purposes intended to be achieved with respect to each of these process parameters.

The free-standing, porous solid or foam PVP-H₂O₂ product also is made by freeze-drying an aqueous solution of PVP powders complexed with an aqueous solution of hydrogen peroxide. The starting PVP polymer material preferably is a water-soluble polymer.

The resulting aqueous solution of PVP and H₂O₂ for freeze-drying suitably contains about 4-10% by weight of H₂O₂, about 15-25% by weight of PVP, and about 65-85% by weight of water. Most preferably, the aqueous solution of PVP and H₂O₂ for freeze-drying contains about 6% H₂O₂, 20% PVP and 74% water.

The aqueous solution thus obtained then is frozen at a temperature of less than -20°C. which forms a soft ice of these materials. The soft ice material then is vacuum dried at these low temperatures under high vacuum, preferably, at about 0.1 mm or less. The water which is removed is condensed in a suitable condenser maintained at about -90°C.

After the freeze-drying step is carried out for some period of time, it is observed that the soft ice is transformed into a free-standing, porous, hard solid which comprises an integral network of PVP molecules hydrogen-bonded to H₂O₂ molecules. The open system in this product result from the loss of water from the interior of the ice body thus providing a porous structure. During transition of the soft ice starting material into the porous, hard network solid, a tacky and porous intermediate product is obtained in which some water is present on the outside of the structure. However, by keeping this material well dispersed and cold, the exterior water content can be readily vacuum-sublimed to provide the advantageous porous and hard solid or foam while avoiding the undesired glassy state.

The final product is a PVP-H₂O₂ complex, which is free-standing and foam-like, and is composed of an integral network of about 70-90% by weight PVP which is hydrogen-bonded to about 10-24% by weight H₂O₂, and where the amount of water present therein is less than 10%. Preferably, the PVP content of this product is about 75-85%, the amount of H₂O₂ is about 15-20%, and its water content is less than about 7-1/2%. Suitably, the total amount of H₂O₂ and water therein is about 25% or less by weight of the product. In the preferred embodiment of the invention, PVP is 77-82%, H₂O₂ is 17-19% and water is about 2-7-1/2%.

In another embodiment of the invention, a water-soluble gelling agent is incorporated within the porous foam during formation to increase its porosity. Suitable gelling agents include hydroxyalkyl cellulose e.g. hydroxyethyl cellulose, propylene and ethylene glycols, polyalkylene glycols, and the like. The resultant porous foam product then comprises about 25-70% PVP, 5-20% H₂O₂, about 20-60% gelling agent; and 0-8% water, by weight of the product.

As a foam, the product has a bulk density of about 0.3 to about 0.5 g/cc and a porosity of about 60-70%.

Of course, if desired, free-flowing powders of such PVP-H₂O₂ products may be obtained by grinding the porous solid.

The invention herein also contemplates the formation of compositions, or mixtures, of the porous solid PVP-H₂O₂ product herein admixed with diluents, carriers, or other active materials. For example, an improved filter material may be provided by forming admixtures of the PVP-H₂O₂ product of the invention with such diluents as inorganic substances such as silica gel, alumina, diatomaceous earth and the like, or organic substances such as polyolefins, polystyrene, polyvinyl chloride and the like, while retaining the desirable pore volume of the porous solid complex. The resultant pore volume of such mixtures for filter application should be between about 0.1 and 2.5 cc per granular of total granular material.

The invention will be illustrated now by reference to the following examples.

### EXAMPLE 1

### PRODUCTION OF PVP-H₂O₂ AS FREE-FLOWING POWDERS USING A FLUIDIZED BED REACTOR AND VACUUM DRYING

0.35 kg of polyvinylpyrrolidone (PVP K-30, CI grade) having particle sizes of predominately 40-50 microns, and a moisture content of 2-3%, was introduced into a 0.4 l fluid bed reactor. The PVP powders were fluidized by passing a stream of dry air upwardly through the charge. The bed temperature was set at 35°-45°C. Then an aqueous solution of 70% H₂O₂ was metered through a spray nozzle with the assistance of an air stream and directed vertically onto the bed. The rate of addition of the solution was 15-25 g of solution/minute/kg PVP for 20 minutes. Adsorption of the solution onto the bed produced a wet product containing 18% H₂O₂ and 6% water. Subsequent downstream vacuum drying of the wet material at 25°-35°C. for 10 hours produced a free-flowing powder which had a 20-22% H₂O₂ content and only 3% water therein.

### EXAMPLE 2

### FREE-FLOWING PVP-H₂O₂ POWDERS USING FLUIDIZED BED DRYER

3.6 kg of polyvinylpyrrolidone (PVP K-30, CI grade), having a particle size of 40-50 microns, was packed into a 22 liter fluidized bed dryer and fluidized at 35°-45°C. using a dry air stream. Then a 70% H₂O₂ solution was introduced during 20 minutes at a feed rate of 10-15 g/minute/kg PVP. During the addition, water was being removed continuously from the product and the bed. Following completion of the H₂O₂ addition, the resultant product was dried further for 10 minutes. The resultant product was a free-flowing powdery complex of PVP-H₂O₂ having a peroxide content of 20% and a moisture content of only 2%.

### EXAMPLE 3

### PVP-H₂O₂ POWDERS USING 30% H₂O₂

The procedure of Example 1 was followed using a 30% H₂O₂ solution at a feed rate of 20 g/minute/kg PVP for 20 minutes. Successive additions of H₂O₂ were followed by vacuum drying. After three stages of application and drying, the free-flowing product contained 19% H₂O₂ and 3% water.

### EXAMPLE 4

### ONE-STAGE PRODUCTION OF PVP-H₂O₂ POWDERS

The procedure of Example 2 was followed using a 50% H₂O₂ solution introduced at a feed rate of 15-22 g/minute/kg PVP. A free-flowing powder product was obtained after addition of the H₂O₂ solution into the fluidized bed dryer. The product contained 18-20% H₂O₂ and 3% water.

### EXAMPLE 5

### PVP-H₂O₂ POWDERS FROM PVP K-90

The procedure of Example 1 was followed using PVP K-90 having a particle size of about 25-75 microns. The free-flowing product contained 17-20% H₂O₂ and 3% water.

### EXAMPLE 6

### PVP-H₂O₂ POWDERS FROM PVP CROSPOVIDONE

The procedure of Example 1 was followed using crosslinked PVP (Crospovidone), which is a water-insoluble PVP, having a particle size of 10-25 microns, with 70% H₂O₂, at a feed rate of 30 g/minute/kg PVP. The free-flowing product contained 18% H₂O₂ and 3% water.

### EXAMPLE 7

### PVP-H₂O₂ POWDERS FROM PVP CROSPOVIDONE USING FLUID BED DRYER

The procedure of Example 6 was followed using a fluid bed dryer, at a H₂O₂ solution feed rate of 13 g/minute/kg PVP. The product contained 18% H₂O₂ and 2% water.

### EXAMPLE 8

### PVP-H₂O₂ POWDERS FROM PVP K-15

The procedure of Example 1 was followed using PVP K-15 having a particle size of 50-80 microns. Two stages of addition and vacuum dyring were required to produce a free-flowing product with a 15% H₂O₂ and 2% water content.

### EXAMPLE 9

### PVP-H₂O₂ AS FREE-STANDING, POROUS SOLID

An aqueous solution of PVP/hydrogen peroxide was prepared by mixing 20 parts by weight of polyvinyl pyrrolidone (PVP K-30) with 80 parts by weight of a 7.6 weight % hydrogen peroxide solution. This solution was then cooled to a temperature below -20°C. whereupon a solid was formed. This solid material was then cryogenically ground while its temperature remained below -20°C. by prechilling a grinder and grinding the frozen solution with dry ice. The granulated material then was charged into a prechilled vacuum jar. A high vacuum of <0.1 mm Hg was applied to the contents while maintaining the jar in a chilled bath at <-20°C. A condenser element was maintained at -95°C. to freeze out any moisture before entering the vacuum pump. After 28 hours, nearly all of the water in the solid was removed leaving porous solid at room temperature containing 15-18% hydrogen peroxide, 3-4-1/2% water and the remainder PVP.

### EXAMPLE 10

### PVP-H₂O₂ AS POROUS FOAM

The procedure of Example 9 was followed except that the aqueous solution was frozen into pieces 2-3 inches thick; then vacuum dried while in the chilled state. The resulting material was foam-like with a bulk density of about 0.43 g/cc and a porosity of 66%. This material had a hydrogen peroxide content of 17-1/2% and a moisture content of 7-1/2% after 48 hours of vacuum drying under the above conditions.

### EXAMPLE 11

90 parts of an aqueous solution of 20% polyvinylpyrrolidone (PVP C-30/CI grade) and 6% aqueous hydrogen peroxide were mixed with 10 parts of hydroxyethyl cellulose (HEC) and allowed to gel for 20 to 30 minutes. The gelled material had an overall composition of: 18.0% PVP, 10.0% HEC, 5.4% H₂O₂, and 66.6% water. The material then was placed in a freeze-dryer at ambient temperature and evacuated to about 0.1 mm Hg for about 18 hours. The resulting solid retained a volume similar to the initial gel, however, the water loss had reduced the sample to 32% of its original weight. Analysis of the product showed a composition as follows: 55.2% PVP, 30.7% HEC, 12.8% H₂O₂ and 1.3% water.

## Claims

1. A process for the production of free-flowing powders of PVP-H₂O₂, optionally including a gelling agent, comprising reacting a fluidized bed of PVP polymer, optionally with a gelling agent, maintained at a reaction temperature of from ambient temperature to 60°C. with finely divided droplets of a 5 to 85% by weight aqueous solution of H₂O₂, and drying the product.

2. A process according to claim 1 wherein introduction of said H₂O₂ solution is continued until the resultant product contains 15 to 24% by weight H₂O₂.

3. A process according to claim 2 wherein said H₂O₂ solution is introduced at a feed rate of 5-50 g/minute/kg PVP.

4. A process according to claim 1 wherein drying is carried out in the fluidized bed during formation of the product, or in a downstream dryer, said reaction and drying steps are carried out in a fluidized bed dryer, or the reaction is carried out in a fluidized bed reactor, and further drying of the wet product is performed in a downstream dryer, optionally wherein air is used to fluidize the PVP powders and to assist in removing water from the fluidized bed during the process.

5. A process according to claim 1 wherein the PVP powders are selected from water-soluble and water-insoluble PVP powders in a range of molecular weights corresponding to the designations K-15 to K-90.

6. A process according to claim 1 wherein the product contains about 5% or less water therein, and said PVP has a particle size of 10-100 microns.

7. A product comprising a PVP-H₂O₂ complex in the form of a free-standing, porous, hard solid or foam having a bulk density of 0.3 to 0.5 g/cc and a porosity of 60-70%, and having an integral network of PVP molecules hydrogen bonded to H₂O₂ molecules, the H₂O₂ content being 10-24% by weight, the water content being less than 10% by weight, and the PVP content being 70-90% by weight, of the product.

8. A process for making the product of claim 7 which comprises:
(a) providing an aqueous solution of H₂O₂ and PVP in the proportions of:
4-10% by weight H₂O₂,
15-25% by weight PVP,
65-85% by weight water; and
up to 60% by weight of a gelling agent,
(b) freezing the solution at below -20°C. to form a soft ice,
(c) vacuum drying at a reduced pressure of less than about 0.1 mm to remove water therefrom.

9. A process according to claim 8 wherein the gelling agent is hydroxyethyl cellulose.

10. A free-standing, porous foam product consisting essentially of an integral network of PVP molecules hydrogen bonded to H₂O₂ molecules, the PVP content being 25-70% by weight, the H₂O₂ content being 5-20% by weight, and including 20-60% by weight of a water-soluble gelling agent to increase the porosity of the foam.

11. A porous foam product according to claim 10 where the PVP content is about 55.2%, the H₂O₂ content is about 12.8%, and the gelling agent is about 30.7%.

12. A porous foam product according to claim 11 which contains less than about 8% water therein.

## Patentansprüche

1. Verfahren zur Herstellung frei fließender PVP-H₂O₂-Pulver, die gegebenenfalls ein Gelierungsmittel enthalten, umfassend das Umsetzen eines Fließbetts aus PVP-Polymer, das gegebenenfalls ein Gelierungsmittel enthält und das bei einer Reaktionstemperatur von Umgebungstemperatur bis 60°C gehalten wird, mit feinverteilten Tröpfchen einer 5 bis 85 Gew.-%-igen wäßrigen Lösung von H₂O₂ sowie das Trocknen des Produkts.

2. Verfahren nach Anspruch 1, worin das Einbringen der H₂O₂-Lösung fortgesetzt wird, bis das resultierende Produkt 15 bis 24 Gew.-% H₂O₂ enthält.

3. Verfahren nach Anspruch 2, worin die H₂O₂-Lösung mit einer Feedrate von 5-50 g/min/kg PVP zugeführt wird.

4. Verfahren nach Anspruch 1, worin das Trocknen im Fließbett während der Bildung des Produkts oder in einem stromabwärts befindlichen Trockner durchgeführt wird, die Schritte des Umsetzens und Trocknens in einem Fließbett-Trockner durchgeführt werden oder die Reaktion in einem Fließbett-Reaktor und weiteres Trocknen des nassen Produkts in einem stromabwärts befindlichen Trockner erfolgt, wobei gegebenenfalls Luft dazu verwendet wird, die PVP-Pulver zum Fließen zu bringen und das Entfernen von Wasser aus dem Fließbett während des Verfahrens zu unterstützen.

5. Verfahren nach Anspruch 1, worin die PVP-Pulver aus wasserlöslichen und wasserunlöslichen PVP-Pulvern in einem den Bezeichnungen K-15 bis K-90 entsprechenden Molekulargewichtsbereich ausgewählt werden.

6. Verfahren nach Anspruch 1, worin das Produkt etwa 5% oder weniger Wasser enthält und das PVP eine Teilchengröße von 10-100 µm aufweist.

7. Produkt, umfassend einen PVP-H₂O₂-Komplex in Form eines frei stehenden, porösen, harten Feststoffs oder Schaums mit einer Rohdichte von 0,3 bis 0,5 g/cm³ und einer Porosität von 60-70% sowie mit einem integrierten Netz aus PVP-Molekülen, die über Wasserstoffbrücken an H₂O₂-Moleküle gebunden sind, wobei der H₂O₂-Gehalt 10-24 Gew.-%, der Wassergehalt weniger als 10 Gew.-% und der PVP-Gehalt 70-90 Gew.-% des Produkts betragen.

8. Verfahren zur Herstellung des Produkts nach Anspruch 7, umfassend:
(a) Bereitstellung einer wäßrigen Lösung von H₂O₂ und PVP in folgenden Anteilen:
4-10 Gew.-% H₂O₂,
15-25 Gew.-% PVP,
65-85 Gew.-% Wasser und
bis zu 60 Gew.-% eines Gelierungsmittels,
(b) Einfrieren der Lösung bei unter -20°C, um ein weiches Eis zu bilden,
(c) Vakuumtrocknen bei einem reduzierten Druck von weniger als etwa 0,1 mm, um Wasser daraus zu entfernen.

9. Verfahren nach Anspruch 8, worin das Gelierungsmittel Hydroxyethylcellulose ist.

10. Frei stehendes, poröses Schaumprodukt, im wesentlichen bestehend aus einem integrierten Netz aus PVP-Molekülen, die über Wasserstoffbrücken an H₂O₂-Moleküle gebunden sind, wobei der PVP-Gehalt 25-70 Gew.-% und der H₂O₂-Gehalt 5-20 Gew.-% betragen, umfassend 20-60 Gew.-% eines wasserlöslichen Gelierungsmittels, um die Porosität des Schaums zu erhöhen.

11. Poröses Schaumprodukt nach Anspruch 10, worin der PVP-Gehalt etwa 55,2%, der H₂O₂-Gehalt etwa 12,8% und jener des Gelierungsmittels etwa 30,7% betragen.

12. Poröses Schaumprodukt nach Anspruch 11, das weniger als etwa 8% Wasser enthält.

## Revendications

1. Procédé pour la production de poudres fluides de PVP-H₂O₂, contenant facultativement un agent gélifiant, comprenant la réaction d'un lit fluidisé d'un polymère de PVP, facultativement avec un agent gélifiant, maintenu à une température de réaction entre la température ambiante et 60°C, avec des gouttelettes finement divisées d'une solution aqueuse à 5 à 85% en poids de H₂O₂ et le séchage du produit.

2. Procédé selon la revendication 1 où l'introduction de ladite solution de H₂O₂ continue jusqu'à ce que le produit résultant contienne 15 à 24% en poids de H₂O₂.

3. Procédé selon la revendication 2 où ladite solution de H₂O₂ est introduite à un débit de 5-50g/minute/kg de PVP.

4. Procédé selon la revendication 1 où le séchage est effectué dans le lit fluidisé pendant la formation du produit ou dans un séchoir en aval, lesdites étapes de réaction et de séchage sont effectuées dans un séchoir à lit fluidisé ou bien la réaction est effectuée dans un réacteur à lit fluidisé, et un plus ample séchage du produit mouillé est accompli dans un séchoir en aval, facultativement où de l'air est utilisé pour fluidiser les poudres de PVP et pour aider à éliminer l'eau du lit fluidisé pendant le procédé.

5. Procédé selon la revendication 1 où les poudres de PVP sont sélectionnées parmi des poudres solubles dans l'eau et insolubles dans l'eau de PVP à des poids moléculaires correspondant aux désignations K-15 à K-90.

6. Procédé selon la revendication 1 où le produit contient environ 5% ou moins d'eau et ledit PVP a une grandeur de particules de 10-100 microns.

7. Produit comprenant un complexe de PVP-H₂O₂ sous la forme d'un solide dur, autonome, poreux ou mousse ayant une densité apparente de 0,3 à 0,5 g/cc et une porosité de 60-70% et ayant un réseau intégral de molécules de PVP qui sont liées par l'hydrogène aux molécules de H₂O₂, la teneur en H₂O₂ étant de 10-24% en poids, la teneur en eau étant inférieure à 10% en poids et la teneur en PVP étant de 70-90% en poids du produit.

8. Procédé de production du produit de la revendication 7 qui consiste à :
(a) prévoir une solution aqueuse de H₂O₂ et de PVP aux proportions de :
4-10% en poids de H₂O₂,
15-25% en poids de PVP,
65-85% en poids d'eau ; et
jusqu'à 60% en poids d'un agent gélifiant,
(b) congeler la solution en dessous de -20°C pour former une glace molle,
(c) sécher sous vide à une pression réduite d'au moins 0,1 mm pour éliminer l'eau.

9. Procédé selon la revendication 8 où l'agent gélifiant est l'hydroxyéthyl cellulose.

10. Produit en mousse poreuse autonome consistant essentiellement en un réseau intégral de molécules de PVP liées par l'hydrogène aux molécules de H₂O₂, la teneur en PVP étant de 25-70% en poids, la teneur en H₂O₂ étant de 5-20% en poids contenant 20-60% en poids d'un agent gélifiant soluble dans l'eau pour augmenter la porosité de la mousse.

11. Produit en mousse poreuse selon la revendication 10 où la teneur en PVP est d'environ 55,2%, la teneur en H₂O₂ est d'environ 12,8% et l'agent gélifiant représente environ 30,7%.

12. Produit en mousse poreuse selon la revendication 11 qui contient moins d'environ 8% d'eau.
